Europäisches Patentamt

European Patent Office

Office européen des brevets

(11) Publication number: **0 431 479 A1**

# EUROPEAN PATENT APPLICATION

(21) Application number: 90122906.2

(51) Int. Cl.⁵: **A61L 15/32, A61L 15/28**

(22) Date of filing: **30.11.90**

(30) Priority: **05.12.89 JP 314401/89**

(43) Date of publication of application:
**12.06.91 Bulletin 91/24**

(84) Designated Contracting States:
**AT BE CH DE DK ES FR GB GR IT LI LU NL SE**

(71) Applicant: **HOECHST JAPAN LIMITED**
**10-16, 8-chome, Akasaka, Minato-ku**
**Tokyo(JP)**

(72) Inventor: **Terao, Toshihiko**
**3776E-122, Handa-cho**
**Hamamatsu-shi, Sizuoka-ken(JP)**
Inventor: **Nin, Hiroshi**
**17-16-601, Nagatsuda 7-chome**
**Midori-ku, Yokohama-shi, Kanagawa-ken(JP)**
Inventor: **Jumonji, Masashi**
**355-270 Shinshukushinden, Showa-machi**
**Kitakatsushika-gun, Saitama-ken(JP)**
Inventor: **Ooi, Fumika**
**22-6-A-704, Tsukuda 2-chome**
**Chuo-ku, Tokyo(JP)**
Inventor: **Kajiwara, Yayoi**
**34-13, Shin-machi 1-chome**
**Setagaya-ku(JP)**

(74) Representative: **Becker, Heinrich Karl**
**Engelbert, Dr.**
**HOECHST AKTIENGESELLSCHAFT Central**
**Patent Department P.O. Box 80 03 20**
**W-6230 Frankfurt am Main 80(DE)**

(54) **Wound surface-covering sheets.**

(57) A combination of collagen and chitin is described providing a covering sheet capable of preventing the adhesion as well as constantly and perfectly accomplishing rapid cure of wounds, in particular, there are provided wound surface-covering sheets comprising a collagen layer and a chitin layer adhered to the upper surface of the former layer.

*Fig. 1*

## WOUND SURFACE-COVERING SHEETS

This invention relates to novel wound surface-covering sheets for covering the surface of wounds, particularly in surgery.

Wound-healing sheets comprising collagen have heretofore been used for the purpose of healing wounds and preventing hemorrhages due to laparotomy. Collagen binds with the platelets exuded from blood vessels to promote platelet aggregation reaction thereby effecting hemostasis. After the hemostasis the collagen is decomposed to gelatin which is inactive in platelet aggregation. As collagen has a fibroblast growth-promoting activity, it also possesses a wound healing-promoting action. Recently, there have been developed lyophilized preparations having the collagen separated and purified from human placenta in the form of sheets, which enable simpler and more convenient use for hemostasis in organs such as the liver and the spleen, myringoplasty, protection of wounds such as spinal injury and imperfect suture of blood vessels.

However, drying on exposure in the laparotomic operation or irritation caused by contact with surgical instruments may produce postoperative adhesion between the wound surface and another organ. Such adhesion is possibly promoted by covering the wound surface with a collagen sheet.

The adhesion is accompanied with pains such as a feeling of traction or feeling of a physical disorder in the abdomen. Under severer conditions it induces disorders of the abdominal tract, ileus and other symptoms. These postoperative disorders in the abdomen occur after surgical elimination of the cause of disease which was done expecting a surgical curative effect. Unfortunately, such disorders induced by postoperative adhesion are increasing year by year in spite of a remarkable progress of modern medicine, and therefore become a serious problem in surgical operations.

Attempts have heretofore been made to prevent adhesion by improving operational techniques, administration of a drug or use of a adhesion inhibiting agent. However, almost none of the preventive measures are satisfactorily effective at present. For example, the adhesion preventing effect by agents currently believed to be effective such as steroids, polyvinyl-pyrrolidone (PVP), chondroitin sulfuric acid or an aqueous solution of sodium alginate is neither constant nor perfect. With a water-soluble adhesion preventing agent the agent may enter normal regions entirely irrelevant to the operation thereby possibly causing rather than preventing adhesion. It is therefore an object of the invention to provide new wound surface-covering sheets having both wound-healing and hemostasis-preventive functions in combination with a function of preventing adhesion to normal regions and other organs.

As a result of extensive studies to achieve the above-mentioned object we have found that combination of collagen and chitin provides a covering sheet capable of preventing the adhesion as well as constantly and perfectly accomplishing rapid cure of the wound. This invention was completed on the basis of said finding.

According to the invention there are provided wound surface-covering sheets comprising a collagen layer and a chitin layer adhered to the upper surface of the former layer.

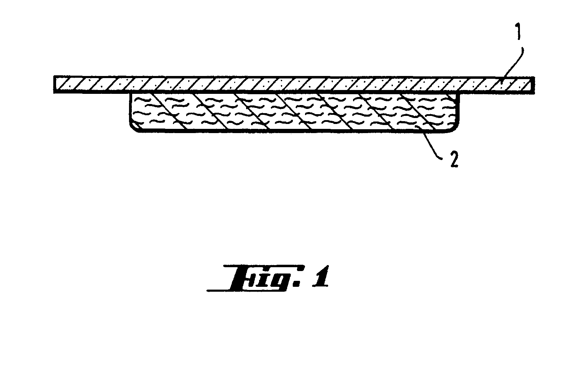

Fig. 1 is a schematic view of the wound surface-covering sheet of the invention.

In Fig. 1, a numeral 1 shows a chitin layer and a numeral 2 shows a collagen layer.

Collagen which is one of the components of the invention is a scleroprotein which is the main component of connective tissues in skin, blood vessels, the stomach, etc. It is also contained in falx, ligament and membrane of the tympanum and is in clinical use as a material for local hemostasis and wound healing (Geka Shinryo (Clinical Practice in Surgery) 31(2), 321, 1989). Collagen is decomposed in vivo to gelatin which is in vivo absorbable.

On the other hand, it has been demonstrated that chitin is also absorbed and decomposed in vivo (Prudden, J.F. et al., Am.J.Surg. 119, 560, 1970).

The chitin layer of the invention is a membrane containing as the main component chitin (poly-N-acetylglucosamine) which is a mucopolysaccharide. The chitin membrane is active in vivo by inducing early consolidation at the site of a skin incision and concurrent inhibition of infiltration of phlogocytes into tissues around the wound. Also, the chitin membrane slightly incorporates the granulation tissues and its in vivo affinity is high. This membrane is effective to promote earlier healing of the wound surface so that it is recognized to be useful as a wound-covering protective agent in skin defects due to burn or trauma (Takehiko Oura et al., Nishinihon Hifuka (Western Japan Dermatology), 50(4), 1988).

The covering sheets of the invention may be formed of integrated layers, one mainly composed of collagen and the other mainly composed of chitin. Each layer may either be of a paper-like dense structure or of a woll-like rough one. Each layer may also be prepared by any method and is not limited to one prepared by a specific method.

As the chitin layer is conveniently employed Beschitin[R] made of short chitin fibers in the form of a non-woven fabric for medical use which is commercially available from Unitika. Density and thickness of the non-woven fabric may appropriately be changed depending upon the site on which they are applied and other conditions. The thickness is usually from 0.1 to 15 mm.

Collagen which is the main component of the other layer is extracted from a source such as human placenta, bovine corium or bovine Achilles tendon. The one of human origin is more preferably and the one extracted from human placenta is most preferably used. These collagens are separated, purified and formed into a collagen film or a collagen sponge which is then processed to a thickness of about 3-10 mm. A collagen layer of fleeced structure is preferred because of its high consolidant effect on the wound surface to a collagen layer of dense structure. The fleeced structure is formed by lyophilizing a collagen solution.

The covering sheets of the invention are prepared by adhering a collagen layer to a chitin layer using a biocompatible adhesive. As the adhesive are used, for example, starch paste, gelatin, chemical synthetic adhesives and fibrinogen. The surfaces may partially be adhered rather than entirely. Both layers may also be stitched up by such a means as surgical suture. The collagen layer may also be adhered to the chitin layer by placing a collagen solution in a dish-like vessel 5-15 mm in depth, superposing a chitin sheet on the solution, freezing the combination, and removing from the vessel and drying under reduced pressure the frozen mass. The covering sheet thus formed is then sterilized by an appropriate means. Covering sheets about 5-10 x 10 cm in size and about 5-10 mm in thickness are frequently used, although the size and the thickness may appropriately be chosen.

The products can be kept at an ordinary temperature or used promptly as needed. The use may be by one application, and peeling-off is not necessary. The covering sheet of the invention can freely be applied by appropriately selecting its area and thickness in accordance with the area of the operation site. The covering sheet is used in such a manner that the collagen layer is in contact with the surface of wounded tissues and the chitin layer is in contact with the surface of other tissues. After being covered, the incision is closed and the components of the covering sheet are assimilated and absorbed in the living body. In addition, a covering sheet with a thinner collagen layer of the invention can also be employed as a wound-covering protective agent by externally applying the chitin layer to the suface of severe burn.

Examples and a test example will be given below to describe the invention in more detail, but the invention is in no way limited by these examples.

Example 1

Frozen human placenta is partially defrozen, pulverized, placed in an aqueous solution of sodium chloride and tetrasodium ethylenediaminetetraacetate, and allowed to stand. The supernatant is then removed to obtain precipitate I. To the precipitate are added water and aprotinin, and the mixture is stirred and then homogenized thereby finely dividing the tissue. The suspension is centrifuged, and the supernatant is removed to obtain precipitate II. To the precipitate II is added a cold sodium chloride solution containing aprotinin, and the micture is stirred. After being allowed to stand, the supernatant is removed. The precipitate thus obtained is washed twice with water followed by addition of a citrate solution and stirring thereby finely dividing the tissue. The suspension is neutralized by addition of a sodium hydroxide solution, and the supernatant is removed to obtain precipitate III. The precipitate III is washed with water and water, acetic acid and pepsin are added to it followed by stirring. After being allowed to stand for 24 hours, additional pepsin is added, and the mixture is stirred and filtered. To the filtrate thus obtained are added water and sodium chloride followed by stirring. The mixture is centrifuged, and the supernatant is removed to give collagen as precipitate IV. To precipitate IV water is added, and the mixture is stirred and homogenized. The resulting collagen suspension is dialyzed to water. After dialysis, the nitrogen content of the suspension is measured, and water is added so as to adjust the nitrogen concentration to 100 mg/g. Formaldehyde is then added as a cross-linking agent (purified human collagen). Tests for HBs antigen and pH measurements are run. Then, the suspension is incubated and filled under laminar flow into a polyethylene plastic vessel. It is then lyophilized, sealed with polyethylene fibers and packed in an aluminum bag coated with polyethylene and polyester. Finally, sterilization is made by gamma-ray irradiation at an absorbed dose of 28 kGy.

As the chitin portion type M of Beschitin[R] W is used (manufactured by Unitika). Beschitin[R] W is made of short chitin fibers processed in the form of a non-woven fabric 0.08-0.5 mm in thickness, which is commercially available as a wound-covering protector.

M (10 cm x 16 cm) Beschitin[R] W (manufactured by Unitika) was cut to a rectangle of 5 cm to 8 cm in size. To one surface of the sheet starch paste was applied (manufactured by Yamoto Nori) in an amount just sufficient to cover it (not excessively). On the surface was mounted a rectangular collagen fleece 5 cm to 8 cm to 0.6 cm in size and thickness in such a way as shown in Fig. 1. The entire sheet wrapped in a thin paper was placed on a flat plate, upon which a similar plate was subsequently superposed. Pressure under a weight of about 200 g was applied to the structure for 10 min.

The weight was then removed, and adhesion of the collagen fleece to the chitin sheet checked. Then, the sheet was allowed to stand overnight at room temperature and placed in a plastic case, which was tightly sealed. Sterilization was done under gamma-rays from cobalt 60 at a minimum adsorbed dose of 25 kGy.

A wound surface-covering sheet of the invention can also be produced, in place of using an adhesive as in the above process, by placing a collagen solution in a dish-like vessel and superposing a chitin sheet upon the solution followed by lyophilization.

Test Example

A 8-week old rat (male, weighing 375 g) anesthetized with pentobarbital is subjected to laparotomy.

After the laparotomy, a portion of the serosa of the large intestine in a size of 0.5 to 0.5 cm is cut out. The wounded surface is covered by a sheet as prepared in example 1 and 1 cm to 1 cm in size, so that the collagen fleece faces the wounded surface. Whereas 50 mg of a synthetic penicillin is intraperitoneally given to prevent bacterial infection in suturing, no agent is used at all for the prevention of adhesion. The test animal is aboserved postoperatively for 15 days for the systemic conditions and state of the faces to check any abnormality. Subsequently, the animal is subjected to euthanasia in a closed vessel in the atmosphere of ether followed by laparotomy to observe the affected part. There occurred cure of the affected part and no adhesion to the circumferential tissues was observed.

## Claims

1. A composition comprising a layer mainly consisting of collagen on which a layer mainly consisting of chitin is adhered.

2. A composition according to claim 1, wherein the chitin layer comprises chitin fibers.

3. A composition according to claim 1, wherein the collagen comprises collagen of human origin.

4. A composition according to claim 1, wherein the collagen comprises collagen from human placenta.

5. A composition according to claim 1, wherein there is a biocompatible adhesive between the two layers.

6. A process for preparing a composition according to claim 1 comprising preparing a collagen layer and a chitin layer and fixing them to each other.

7. A process according to claim 6 wherein the layers are fixed to each other with a biocompatible adhesive.

8. A method of use wherein a composition according to claim 1 is used in a process for preparing a wound-covering material.

Claims: ES, GR

1. A process for preparing a composition comprising preparing a collagen layer and a chitin layer and fixing them to each other.

2. A process according to claim 1 wherein the layers are fixed to each other with a biocompatible adhesive.

3. A process according to claim 1, wherein the chitin layer comprises chitin fibers.

4. A process according to claim 1, wherein the collagen comprises collagen of human origin.

5. A process according to claim 1, wherein the collagen comprises collagen from human placenta.

_Fig. 1_

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-3 800 792 (J.J. McKNIGHT)<br>– – – | | A 61 L<br>15/32<br>A 61 L 15/28 |
| A | EP-A-0 295 777 (JAPAN GORE-TEX)<br>– – – – – | | |

|  |
|---|
| TECHNICAL FIELDS SEARCHED (Int. Cl.5) |
| A 61 L 15/32<br>A 61 L 15/28 |

The present search report has been drawn up for all claims

| Place of search | Date of completion of search | Examiner |
|---|---|---|
| The Hague | 27 February 91 | PELTRE CHR. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document